# EUROPEAN PATENT APPLICATION

(11) **EP 2 842 511 A1**
(43) Date of publication of application: **04.03.2015**
(21) Application number: 14182120.7
(22) Date of filing: 25.08.2014
(51) Int. Cl.: A61B 19/00, A61B 17/34, A61B 1/313, A61B 1/00

(54) **Method for minimally invasive surgery stereoscopic visualization**

(30) Priority: 27.08.2013 US 201314011510
(71) Applicant: Vantage Surgical Systems, Inc., Irvine, CA 92618 (US)
(72) Inventor: Wilson, Jason, Santa Ana, CA 92704 (US); Arat, Vacit, La Canada Flintridge, CA 91011 (US); Blumenkranz, Mark, Portola Valley, CA 94028 (US)
(74) Representative: Hirsch & Associés

(57) **Abstract**

Aspects of the present invention provide improved visualization systems and methods for minimally invasive surgery. Some embodiments include the use of reverse kinematic positioning of camera systems to provide rapid and manual surgeon controllable positioning of camera systems as well as display of 3D surgical area images along the line of sight between a surgeon's eyes and the surgical area itself.

## Description

### RELATED APPLICATIONS

This application claims benefit of United States Provisional Patent Application No. 61/693,551 filed August 27, 2012, and is a Continuation in Part of United States Non-Provisional Patent Application No. 13/761,136 filed February 6, 2013 which claims priority to United States Provisional Patent Application 61/595,467 filed February 6, 2012. Each of these referenced applications is incorporated herein by reference as if set forth in full herein.

### FIELD OF THE INVENTION

The present invention relates generally to the field of minimally invasive surgery (MIS) and more particularly to improved visualization methods and tools for use in such surgical procedures.

### BACKGROUND OF THE INVENTION

During minimally invasive surgical procedures it is common for hand held endoscopes to be used for visualization where images captured by these endoscopes are displayed on monitors that are placed away from the surgical field.

In this configuration the surgeon has given up control to an assistant (assistant surgeon, attending nurse, etc.) to steer the endoscope under his/her verbal instructions. To achieve high quality magnified views of the surgical field, optical zooming is performed by physically moving the endoscope closer to the field by the assistant. Digital zoom is also an option; however, this approach suffers from reduced pixel resolution. Image quality is also a function of the endoscope objective aperture size, and it is exacerbated for stereoscopic endoscopes as there is a requirement for two objectives at the distal end for the same size diameter.

Furthermore, compared to open surgery, the entire experience of viewing the surgical field is unintuitive and ergonomically incorrect on many levels. While in open surgery the surgeon looks at where the practitioner's hands and instruments are and work in line with his/her visual axis, in MIS the practitioner looks at a direction unrelated to his/her visual axis. In most cases, the practitioner also gives up 3-D views with full depth perception and peripheral vision which allows views of the surgical tools. In addition, the practitioner's eyes are accommodated to a distance 4-5 times further than the patient - exacerbating the connection in his/her brain between the views and the work being performed.

### SUMMARY OF THE INVENTION

The foregoing needs are met, to a great extent, by the present invention, wherein in some aspects of embodiments of the invention are intended to address one or more of the above noted fundamental problems associated with visualization systems used in conventional minimally invasive surgery. The Improved visualization methods and system of the various embodiments of the invention are applicable to many types of minimally invasive surgery, for example in the areas of thoracoscopic, laparoscopic, pelviscopic, arthroscopic surgeries. For laparoscopic surgery, significant utility will be found in cholecystectomy, hernia repair, bariatric procedures (bypass, banding, sleeve, or the like), bowel resection, hysterectomy, appendectomy, gastric/anti-reflux procedures, and nephrectomy.

In some aspects of the disclosure one or more of these problems are addressed by returning control of a stereoscopic video camera to the surgeon via a novel steering frame. The stereoscopic video camera can be able to obtain stereoscopic images via a single objective lens thus allowing for more light and higher spatial resolution. In some embodiments, the stereoscopic monitor may be moved to an ergonomically correct location while allowing for direct line of sight positioning of the stereoscopic camera and autostereoscopic (glasses-less) 3D visualization. In addition, an ancillary benefit of the monitor repositioning can be a larger field of view for the surgeon performing the MIS.

In a first aspect of the invention a method for viewing a surgical area of a minimally invasive surgical procedure is disclosed. The method including: capturing an image of an internal surgical area via an image capturing device including at least two optical paths, at least one objective lens assembly inserted into a retaining plug positioned through a percutaneous incision, and an end effector forming part of a frame supporting the image capturing device; configuring said end effector to be movably coupled with respect to the internal surgical area; further configuring a locking mechanism to lock said end effector at a desired position relative to the internal surgical area; transmitting said captured image to a display positioned to be viewed by a practitioner during a minimally invasive surgery; and processing a practitioner's input to modify the image being displayed.

According to some aspects of the disclosure, the method can include: capturing an image of an internal surgical area via an image capturing device including two optical paths, an objective lens assembly including retaining structures useful to hold the objective lens assembly inside a percutaneous incision, and an end effector forming part of a frame supporting the image capturing device; configuring said end effector to be movably coupled with respect to the internal surgical area; further configuring a locking mechanism to lock said end effector at a desired position relative to the surgical area; transmitting said captured image to a display positioned in a sterile field to be viewed by a practitioner during a minimally invasive surgery; and processing a practitioner's input to modify the image being displayed.

According to other aspects of the disclosure, the method includes: capturing an image of an internal surgical area via an image capturing device including an objective lens assembly with a proximal end, a distal end, and one or more optical lenses in between, inserted at least partially into a percutaneous incision, and an end effector forming part of a frame supporting the image capturing device, wherein said objective lens assembly can be placed such that the proximal end is outside of the patient's body white the distal end is disposed inside of the body cavity; and configuring said end effector to be movably coupled with respect to the internal surgical area; further configuring a locking mechanism to lock said end effector at a desired position relative to the surgical area; and transmitting said captured image to a display positioned in a sterile field to be viewed by a practitioner during a minimally invasive surgery.

In yet additional aspects of the disclosure, locking mechanisms can include one or more of a mechanical, pneumatic, and electrical locking mechanism. In some embodiments, configuring said retaining plug and/or the objective lens assembly to include a light source capable of illuminating the internal surgical area can also be useful. The captured image/video may be processed to provide three-dimensional images/views. Further, a practitioner's commend can be processed to change the magnification and/or rotational view of said captured stereoscopic image/video.

Other aspects of the invention will be understood by those of skill in the art upon review of the teachings herein. Other aspects of the invention may involve combinations of the above noted aspects of the invention. These other aspects of the invention may provide various combinations of the aspects presented above as well as provide other configurations, structures, functional relationships, and processes that have not been specifically set forth above.

### BRIEF DESCRIPTION OF THE DRAWINGS

FIG. 1 provides a perspective view of a stereoscopic image capture system including a stereoscopic video camera assembly (e.g. a stereoscopic digital video acquisition unit and minimally invasive objective lens assembly which may be referred herein simply as an objective), a steering frame, and a minimally invasive retaining plug into which the objective lens assembly fits for use in a minimally invasive surgery/procedure according to a first embodiment of the invention;
FIG. 2 provides a cross-sectioned perspective view of the stereoscopic image capture system of FIG. 1;
FIG. 3 provides a side view of the stereoscopic image capture system of FIG. 1 while the plug and lens stack penetrate a patient's abdominal wall at a 12.5° viewing angle;
FIG. 4 provides a close up cross sectioned perspective view of the interaction between steering frame, the minimally invasive retaining plug and wide angle objective lens stack while the plug and lens stack extend through a patient's external tissue;
FIGS. 5A and 5B provide a sectioned perspective view and a non-sectioned perspective view, respectively, of one of the prismatic struts and an associated locking mechanism that joins the proximal and distal ends of the steering frame to one another in a movable manner;
FIG. 6 provides a perspective view of the locking mechanism shown in FIG. 5B;
FIG. 7 provides a view of a patient's body, the stereoscopic image capture system of FIG. 1, and view of a surgical area as displayed on a viewing screen located in proximity to the actual surgical area from the vantage point of a surgeon during a minimally invasive surgical/procedure according to a procedural embodiment of the invention;
FIG. 8A shows the geometric effect which makes the field of view from the human eye of a 5.5 inch monitor at a distance of 2 feet identical to that of a 22 inch monitor at 8 feet while FIG 8B shows the same geometric effect which allows a monitor that is 11 inches to provide a larger field of view at the same working distance;
FIG. 9A provides a perspective view of an actuation interface for the passive steering frame according to an implementation of the first embodiment;
FIG 9B provides a close up, cross sectioned perspective view of an actuation mechanism for the passive steering frame;
FIG 10 provides an isometric view of the distal end of the objective assembly and plug showing an embedded a ring of LED illumination devices forming part of the plug according to some aspects of the disclosure;
FIG 11 provides a block diagram showing the mechanical interconnections between a patient and the components of an image capture and display system of an embodiment of the disclosure as used during a minimally invasive surgical procedure or visualization procedure;
FIG 12 is a block diagram showing alternative mechanical interconnections between a patient and the components of an image capture and display system of an embodiment of the invention as used during a minimally invasive surgical procedure or visualization procedure;
FIG. 13 is a block diagram showing alternative electrical and mechanical interconnections between a patient and the components of an image capture display of an embodiment of the invention as used during a minimally invasive surgical procedure or visualization procedure;
FIG. 14 provides a perspective view of an alternative configuration for the steering frame that allows the base to attach the prismatic joints to the patient or table using a plurality of base pads or feet;
FIG. 15 provides a perspective view of another alternative configuration where the steering frame is attached to an external support device;
FIG. 16 provides a perspective view of another alternative configuration of the steering frame where it is a serial manipulator rather than a parallel manipulator;
FIG. 17A provides an exemplary view of a surgical display showing how the display can show two images of the surgical field in a picture-in-picture format with each image showing a large zoomed view and inlaid wide angle view;
FIG. 17B provides an exemplary view of a surgical display showing how the display can show two images of the surgical field in a picture-in-picture format with each image showing a small view and zoomed in inlaid wide angle view;
FIG. 18A provides an example view of the surgical display showing how image processing can compensate for orientation errors or variations caused by misalignment of the imaging device relative to the viewing direction. In particular FIG. 18A shows a first image from a viewing perspective that is different from the viewing perspective of the surgeon relative to the patient's body;
FIG. 18B provides an example view of the surgical display showing how image processing can compensate for orientation errors or variations caused by misalignment of the imaging device relative to the viewing direction. In particular, FIG. 18B shows the transformed perspective such that the displayed image is matched to the surgeon' viewing direction;
FIG. 19 provides an example view of the operating theater wherein the surgical display is a touchscreen computer that can display the video stream from the camera, and communicate with the camera, external monitors, external computers, external medical devices, and other peripheral electronic equipment;
FIG. 20 provides an example view of the surgical field wherein the display is a touchscreen computer wherein touchscreen inputs are translated into commands which steer the camera automatically;
FIG. 21 provides an example cross section view of a lighting apparatus that is compact while being inserted through the abdominal wall wherein it expands upon entering a surgical cavity;
FIG. 22 provides a block diagram showing alternative interconnections between components in which the drive electronics to the steering arm are taking commands from the touch screen computer display; and
FIG. 23 provides a flowchart illustrating exemplary methods steps that can be implemented according to aspects of the present disclosure.

### DETAILED DESCRIPTION OF THE INVENTION

The disclosure will now be described with reference to the drawing figures, in which like reference numbers refer to like parts throughout. Various aspects of the invention may be illustrated by components that are coupled, sealed, attached, and/or joined together. As used herein, the terms "coupled", "sealed", "attached", and/or "joined" are used to indicate either a direct connection between two components or, where appropriate, an indirect connection to one another through intervening or intermediate components. In contrast, when a component is referred to as being "directly coupled", "directly sealed", "directly attached", and/or "directly joined" to another component, there are no intervening elements present.

Relative terms such as "lower" or "bottom" and "upper" or "top" may be used herein to describe one element's relationship to another element illustrated in the drawings. It will be understood that relative terms are intended to encompass different orientations in addition to the orientation depicted in the drawings. By way of example, if aspects of exemplary embodiments shown in the drawings are turned over, elements described as being on the "bottom" side of the other elements would then be oriented on the "top" side of the other elements. The term "bottom" can therefore encompass both an orientation of "bottom" and "top" depending on the particular orientation of the apparatus.

Various aspects of the stereoscopic systems for minimally invasive surgery visualization are illustrated with reference to one or more exemplary embodiments. As used herein, the term "exemplary" means "serving as an example, instance, or illustration," and should not necessarily be construed as preferred or advantageous over other embodiments disclosed herein.

According to aspects of the disclosure, control of an image capturing devise for minimally invasive surgery (MIS) procedures can be returned to the surgeon via a novel steering frame. In some embodiments, the image capturing device can be a stereoscopic video camera that is able to obtain stereoscopic images via a single objective lens thus allowing for more light and higher spatial resolution. In some embodiments, a stereoscopic monitor can be moved to an ergonomically correct location while allowing for direct line of sight positioning of the stereoscopic camera and autostereoscopic (glasses-less) 3D visualization. In some embodiments, an ancillary benefit of the monitor repositioning is a larger field of view.

MIS procedures that can implement system aspects disclosed can include, for example, in the areas of thoracoscopic, laparoscopic, pelviscopic, arthroscopic surgeries. For laparoscopic surgeries for example, significant utility will be found in cholecystectomy, hernia repair, bariatric procedures (bypass, banding, sleeve, or the like), bowel resection, hysterectomy, appendectomy, gastric/anti-reflux procedures, and nephrectomy.

Some advantages of one or more aspects of the present disclosure can include:
(1) Intuitive visualization: Unlike an endoscope, since the stereoscopic video camera is able to zoom optically without any external physical movement, the total occupied space can be significantly smaller. This coupled with the repositioning of the display allows the surgeon to perceive the surgical field close to what he/she would have experienced in open surgery. Furthermore, since the display can be at the appropriate distance from the surgeon (i.e. it is at approximately the same distance as the organs during open surgery) the accommodation of the eye can be ergonomically correct; (2) Return of visual control to the surgeon: Because of the novel steering frame, the surgeon is able to steer the camera to look at the portion of the surgical field that is desired without use an attending nurse. Zoom can be controlled by the surgeon as well since it is optical rather than by physically moving the camera/endoscope; (3) Optical Zoom: Endoscopes don't have optical zoom. Zooming is done by moving the distal end of the endoscope closer to the target or digitally. Both approaches result in a degradation of image quality from either lack of light or reduced pixel resolution; (4) Superior Optics: since the objective lens assembly need only be one lens rather than two, it can be bigger resulting in a large objective aperture, allowing for more light and better spatial resolution while still obtaining a stereoscopic image; (5) Panoramic view: The objective lens assembly can be made such that there is a very wide viewing angle (as much as 90 degrees) allowing for easy surgical instrument visualization. This is a big problem associated with endoscopes as there distal ends are typically positioned very close to the anatomy undergoing surgery; and (6) Passive steering frame: In order to allow the stereoscopic video camera to be positioned arbitrarily relative to the surgical incision, a passive steering frame is preferred. Specifically it differs from other structural frames as it is moved in an inverse kinematic modality. Instead of adjusting "joint angles" to realize the correct end effector location (forward kinematics), the end effector is moved to the location and then maintained by locking the joints at their natural position (inverse kinematics) as will be explained in more detail hereafter.

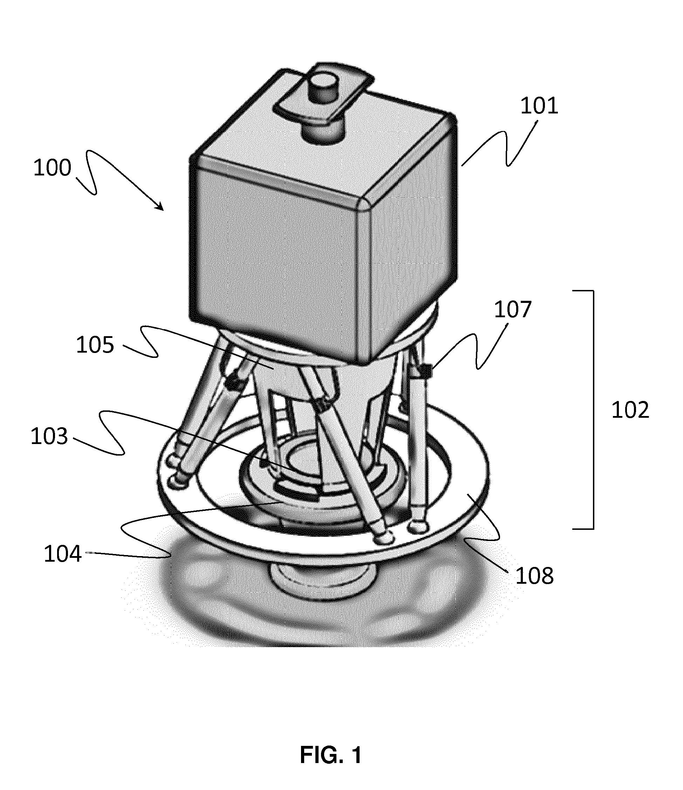

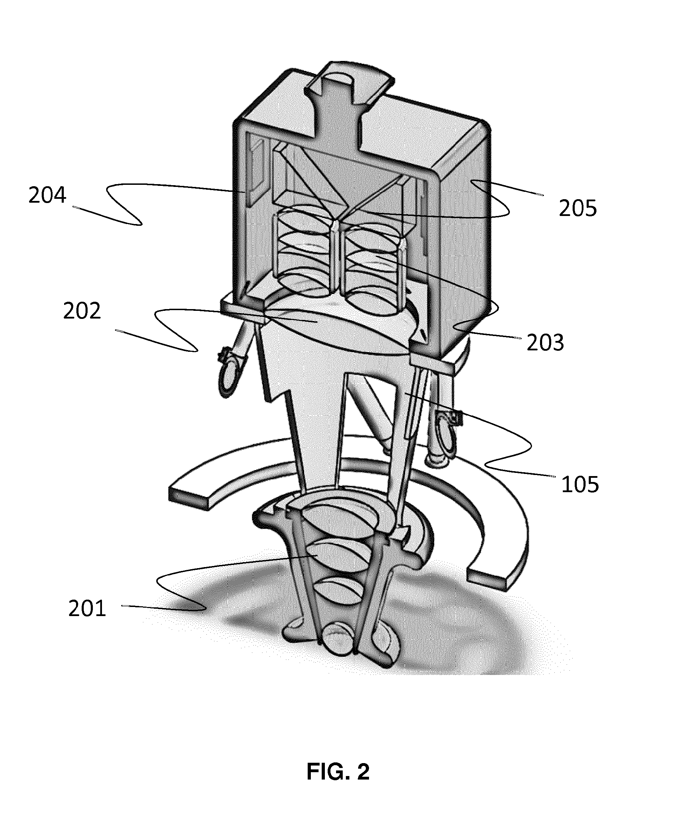

Referring now to FIG. 1 and FIG. 2, perspective views of a stereoscopic video camera and steering frame assembly 100 are illustrated. The camera can include two components - a stereoscopic digital video acquisition unit 101 and minimally invasive objective lens assembly 103. The internal components of the objective 103 include a lens stack (as shown in FIG. 2) which allows for a wide angle view of a surgical scene through a typical minimally invasive surgical incision. The video acquisition unit 101 can include a number of internal components including a magnification lens 202, two stereoscopic pupils 203 with zoom and focus capabilities, optical path diversion components 205 (e.g. mirrors or prisms), and at least two photosensitive integrated circuits 204 (the left component can be seen in FIG. 2) for image digitization. The optical path of the video acquisition unit 101 and objective 103 can be kept optically aligned by a structural coupler 105. This stereoscopic video camera and steering frame assembly 100 (including the video acquisition unit 101, the structural coupler 105 and the objective 103) may be attached to a passive steering frame 102. The frame 102 can comprise locks 107 that allow the frame 102 to transition from a movable state to a rigid state. When the locks 107 are off, the frame may be able to be moved manually to allow for the correct positioning (e.g. height and orientation) of the assembly 101, 105,103. When the locks 107 are engaged, the frame 102 can be rigid and thus free standing, allowing the surgeon to let go of the device 100. As the objective 103 may lift out of the incision, in some embodiments it may be desired to include a retaining plug 104 where the objective 103 can be inserted into to prevent it from coming out of the incision.

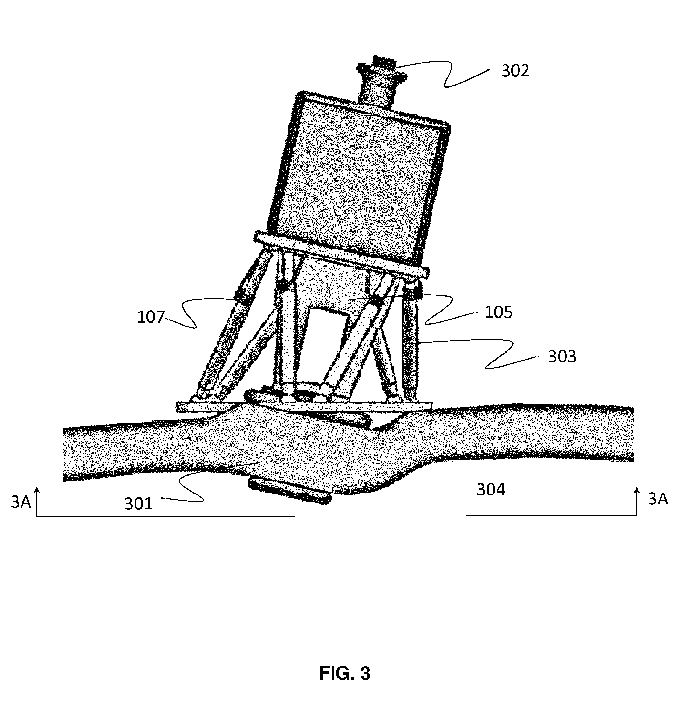

Referring now to FIG. 3, the device or assembly 100 as it is inserted through a minimally invasive surgical incision that can extend through the layers of skin 301 and penetrates into an insufflated cavity 304 is depicted. The cavity may be insufflated before or after insertion of the assembly 100 depending on whether or not adequate sealing of the cavity can be possible before insertion occurs (e.g. via a pre-inserted plug). According to some aspects of the disclosure, the frame 102 can include a hexapod that can allow for six degrees of freedom to move the optical path as it may be desired. For example, as depicted with the optical path displaced 12.5o off center. This displacement can be facilitated by manually releasing the locks 107, and manually rotating the device 100 via a steering handle 302 that, for example, can be located at the top of the camera assembly (as shown) and that may be grasped while pushing a release button located at the top of the handle (as shown). The button may release the locks that retain the plurality of struts 303 at fixed lengths. The released locks in turn allow the struts to change their respective lengths (e.g. by low friction sliding) to adjust to their lengths to correspond to a new position to which the surgeon locates the camera assembly. This lock release and assembly repositioning can result in extension and compression of the appropriate struts 303 and then release of the button allows for reengagement of the locks and thus fixed strut 303 positioning the optical path in a desired angle.

In some alternative embodiments, the push button may be replaced by another mechanism (e.g. another type of switch such as a lever or a slider that actuates a spring loaded mechanical release mechanism, a bistable mechanical release and lock mechanism, an electrical, magnetic, pneumatic or hydraulic lock and/or release mechanism. In such alternative embodiments the switch or slider may cause locking in one position and release in another. In still other embodiments, the multiple buttons switches or sliders may be integrated into the steering handle 302 such multiple switches may be used to lock or release different struts 33 (e.g. to allow limited repositioning along different axes.

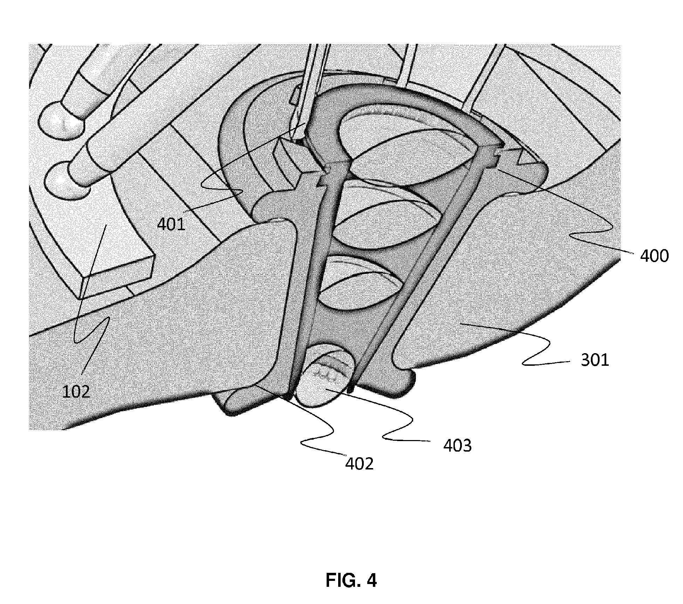

Referring now to FIG. 4, a perspective view of the assembly shown in FIG. 3 through line 3A showing the interconnections between the steering frame 102 the objective 103 and the retaining plug 104 is depicted. The frame 102 may sit flush on the outer surface of the patient's skin 301. In some alternatives, the frame may sit flush against the patient's skin but be supported by other structural extensions such as a ceiling, wall, or stand supported arm or arms or by structural elements that extend beyond the patient's body to a table. In yet additional alternatives, the base may be positioned on a pad, sterile sheet protector, or the such, resting on a patient's skin 301.

The structural coupler 105 (shown in FIG. 1) can be connected to the top portion of the steering frame 102 which can be attached directly to the video acquisition unit 101. This connection may be permanent such that the structural coupler 105 can be merely a component of the steering frame 102, or is otherwise rigidly attached (though in a detachable manner) so as not to flex under stress, or designed to flex only after the amount of force is greater than a surgeon would typically apply to move the optical path is applied (therefore flexing to prevent organ damage). The structural coupler 105 can be directly attached (e.g. permanently or detachably) to the objective 103 creating a rigid body connection. The objective 103 can also be attached to the retaining plug 104 creating another rigid body connection. The retaining plug 104 can preferably include a flared distal end 402 that can extend underneath the skin layer(s) 301. Additional information about retaining plugs 104 and functional relationships between retaining plugs 104 and lens assemblies 103 is found in US Patent Application No. 13/268,071, filed 10/7/2011 (VSSP-009US-A) which is incorporated herein by reference as if set forth in full herein. In some alternative embodiments, the lens assembly 103 may not be directly coupled to the structural coupler 105 but instead may be directly coupled to the retaining plug 104 and the retaining plug 104 directly coupled to the structural coupler 105.

In the present exemplary embodiment, the retaining plug 104 is flexible, and in the absence of the objective lens assembly 103, it can be compressed or bent such that it can pass through the incision (e.g. by folding). Once the retaining plug 104 is positioned, the objective 103 can be inserted such that when it is seated, a retaining feature 400 attaches to the proximal end of the objective 103. Similarly, when the frame 102 is positioned to rest on the external surface of the skin, the structural coupler 105 may be moved such that the retaining feature 401 at the distal end of the coupler attaches to the proximal end of the objective 103. By nature of this interconnection, when the steering frame 102 pushes down on the skin 301, an opposite force can be applied to the objective 103 tending to remove it from the incision. However while connected to the objective 103 the retaining plug 104 is unable to move via the flared distal end 402 applying light tension to the skin 301. This can securely seat the stereoscopic video camera 101 (shown in FIG. 1) and steering frame 100 to the body of the patient.

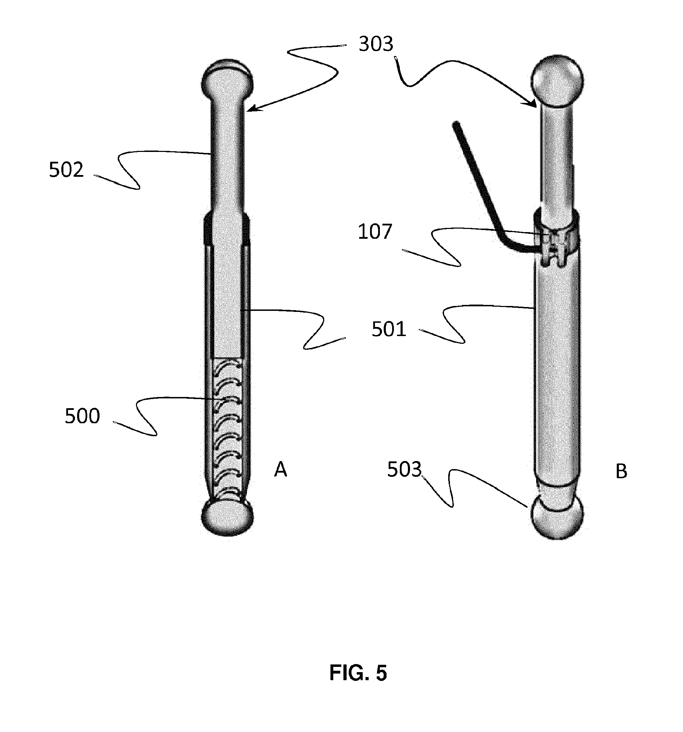

Referring now to FIG. 5A, a cross sectioned perspective view of an exemplary steering frame strut 303 is depicted. In particular, the steering frame strut 303 including a prismatic joint with two sections. In this exemplary embodiment, the prismatic joint consists of a male component 502 and a female component 501 that can move relative to each other in a prismatic fashion. In order to ensure pressing of the base of the frame against the skin 301 (at any given position), a spring mechanism 500 may be added to the strut 303 to make it tend towards expansion. In some alternative embodiments, the biasing spring may be removed in favor of a base that seats against the skin by its weight. In other embodiments, it may be replaced a constant pressure pneumatic cylinder or similar device known in the art.

Referring now to FIG. 5B, an external perspective view of a steering frame strut 303 is depicted. In particular, the joints 503 at the top and bottom of the strut may be useful to facilitate at least two rotational degrees of freedom orthogonal to the axis of the strut 303. This can be typically achieved using a universal or ball joint. In this exemplary embodiment, the lock 107 is also shown attached to the female component 501.

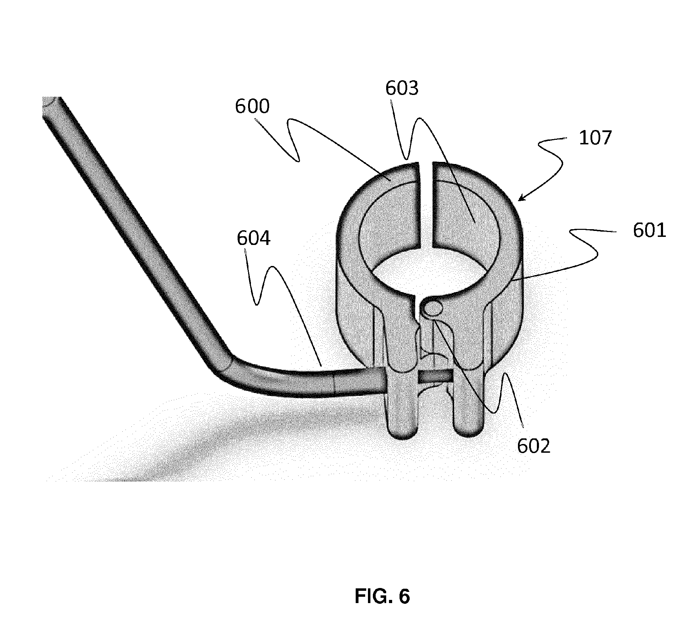

Referring now to FIG. 6, a perspective view of an exemplary embodiment of a mechanical locking mechanism is depicted. In particular, the mechanical locking mechanism 107 which can be comprised of two independent arms 600 & 601. The arms 600 & 601 can be connected to a joint 602 which can be in turn connected to the female component 501 of the strut 303. The joint 602 should tend to close the lock 107 when no external forces are applied by the actuation mechanism 604. Such lock biasing may be achieved in a number of different ways, for example, by locating a compression spring or elastic material between the arm extensions of the lock 107 and/or a tensioned spring (not shown) connecting the open ends of the arms 600 & 601. The mounting configuration of the lock 107 on the female component 501 can be such that, when closed, the surface 603 can come in contact with the male component 502. This mechanical interaction (either friction or mechanical interference) can be such that the strut becomes a rigid body. When desired to unlock the strut components, actuation mechanism 604 can apply a force sufficient to overcome any seating force that clamps the lock arms 600 & 601 together. This actuation force may take a variety of forms such as mechanical (e.g. via a tensioned wire), pneumatic, electrical and/or pneumatic.

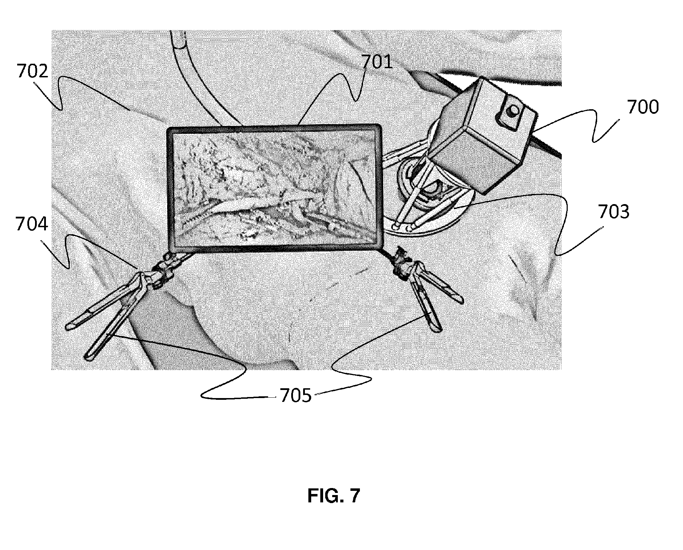

Referring to FIG. 7, a view of the Stereoscopic Video Camera, Steering frame and Display for Minimally Invasive Surgery Visualization as seen from the vantage point of the surgeon is illustrated. The stereoscopic video camera 700 and steering frame 703 are seen resting on the patient 702 with the objective 103 and retaining plug 104 inserted in an incision. The display 701 can be placed in front of the surgeon such that it can be oriented to reflect what the direct line of sight of the surgeon would be. Shown in alignment, on the monitor and outside the body, are two surgical tools 704 and their operational handles 705. In one embodiment, the display 701 may be a full high definition 1920x1080 progressive 3D monitor of the type that does not require glasses (e.g. parallax barrier). In other embodiments, other 2D or 3D displays may be used.

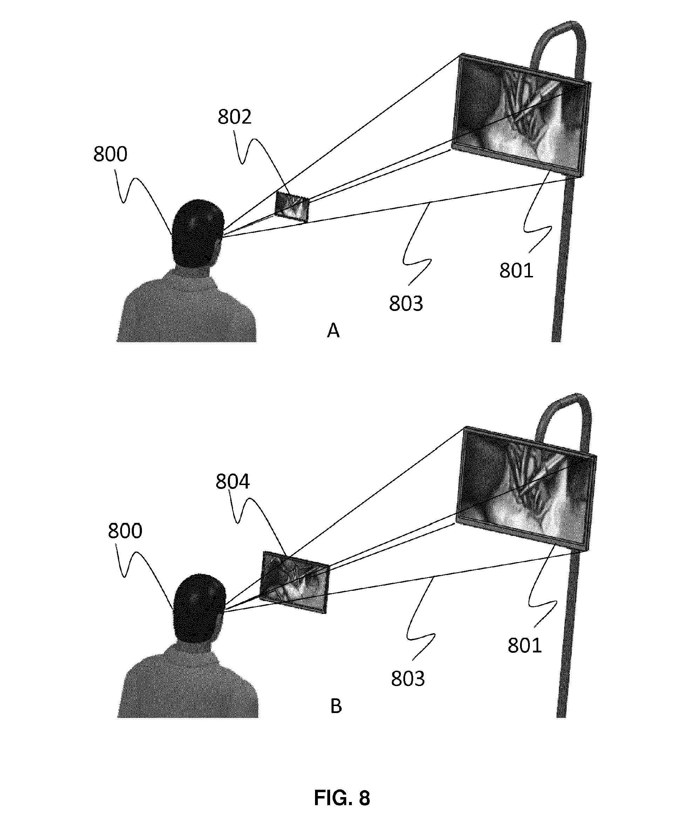

Referring now to FIG. 8A, the perspective equivalence of a surgeon 800 viewing two displays at different distances with different sizes is depicted. In particular, a 22 inch display 801 is shown as being viewed at a distance of 8 feet which is typical for standard surgical theater configurations. A 5.5 inch display 802 is shown as being viewed at a distance of 2 feet from the surgeon. The equivalence of these views is illustrated by the perspective view rays 803 originating from the light of sight of the surgeon 800 and intersecting the four corners of both monitors 801 & 802. In comparison FIG. 8B is an illustration of the same perspective view rays 803 as seen in FIG. 8A, however with the 5.5 inch display 801 replaced with an 11 inch display 804. The result is that a small display 804 can provide a much larger field of view at the correct eye accommodation distance.

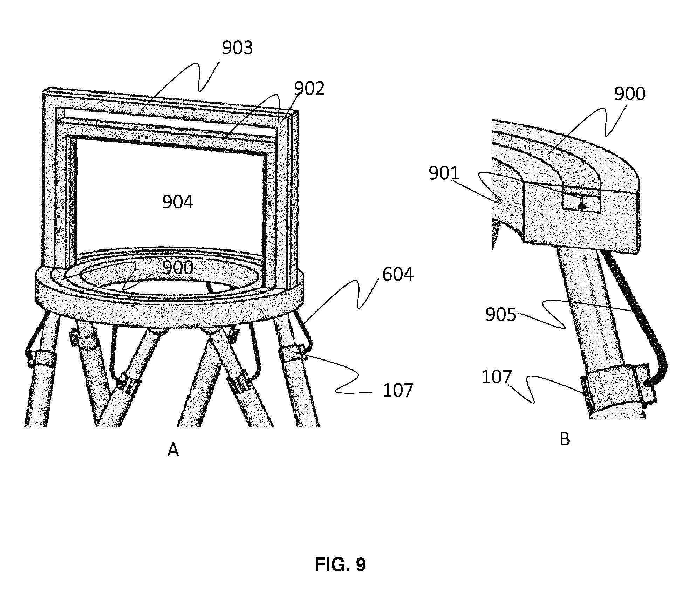

Referring now to FIG. 9A, another exemplary interface for the lock actuation mechanism for use with a steerable frame wherein a handle is provided instead of a push button is illustrated. In particular, the interface can consist of a steering handle 903 (as opposed to the knob-like handle of FIGS. 1 and 2) and a lock activation/deactivation switch 902. The switch 902 can be coupled to all of the actuation mechanisms 604 associated with each strut 303 via an actuation coupling mechanism 900 which can be in the form of a ring (see the discussion below concerning FIG. 9B). The lock activation/deactivation switch 902 can have at least two positions, one position that can activate all locks 107 (e.g. a down position that causes all lock mechanisms to engage the male portion of their respective struts), and another position that can deactivate all locks 107 (e.g. an up position that pulls the tensioning wires that cause the back ends of the lock arms to move together thus opening the arms or jaws of the lock that provide a clamping function). The geometry of the steering handle 903 and switch can be such that there is space 904 to attach the object to be steered - in this case the stereoscopic video camera 101.

Referring now to FIG. 9B, a close up perspective view of the actuation coupling mechanism 900 and the associated actuation mechanism 901 is illustrated. In this embodiment, the actuation mechanism 901 can be cable based, where a cable 901 can be pulled through a sheath 905 that is capable of actuating the lock 107. The sheath 905 surface 603 pushes on one lock arm while the cable 901 pulls on the other 901, thus deactivating the lock 107 much like a bicycle brake. In this embodiment the coupling mechanism can be a disk that attaches to all six cables 901 simultaneously which may in turn be substantially connected to the lock activation/deactivation switch 902.

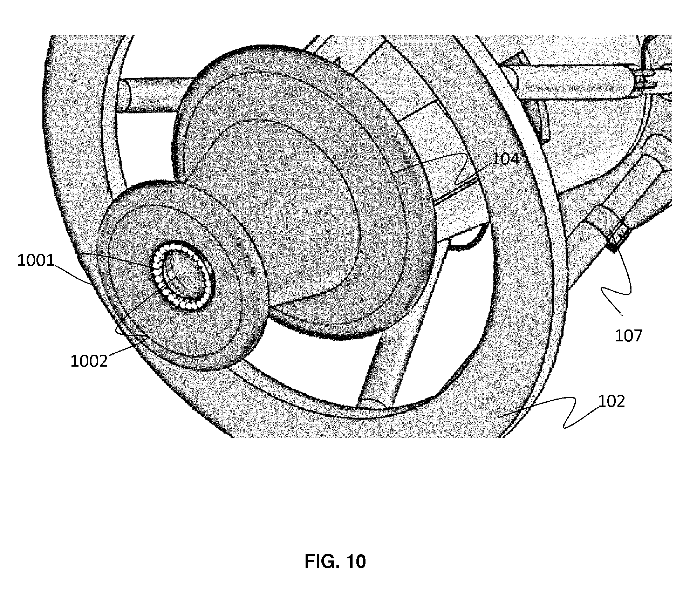

Referring now to FIG. 10, an isometric view as seen looking at the distal end of the objective lens assembly 103 and retaining plug 104 is depicted. In order to illuminate the scene, in this embodiment, LED lights 1001 are placed at the distal end of the objective 103 or the retaining plug 104. Due to the large aperture 1002 of the objective 103 - which can be approximately 25 times larger by area than a two objective 3D endoscope - the lighting does not need to be as bright as the current standard practice of xenon based illumination. This can allow LED lighting to be adequate for MIS. In other embodiments, instead of LED lighting, fiber optics may be provided, as part of the lens assembly or plug, to direct light from an external source into the surgical area. In still other embodiments, light may be brought to the surgical area via one or more additional incisions.

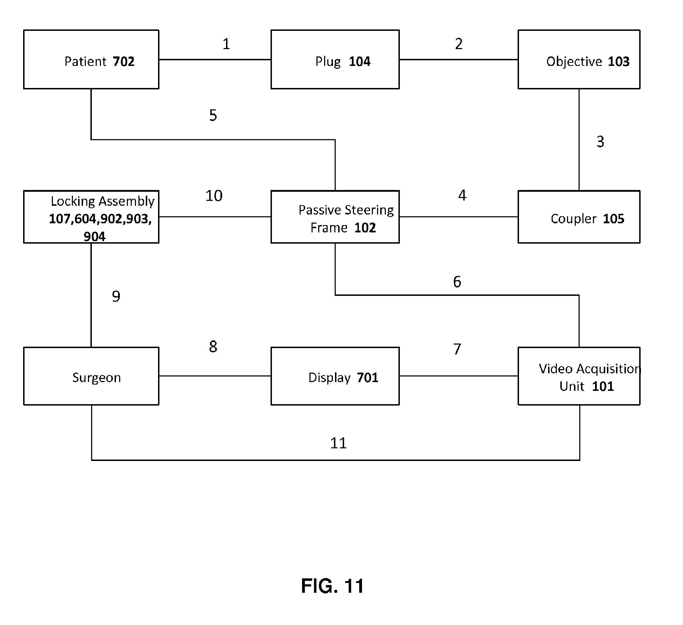

Referring now to FIG. 11, a block diagram showing the mechanical and electrical interconnections between a patient and the components of an image capture and display system of an exemplary embodiment of the invention as used during a minimally invasive surgical procedure or visualization procedure is depicted. Each physical component in the block diagram is designated by a rectangle with the description of it within. The interaction between two connected components is designated by a line and each line is provided with a reference number that is described.

In this exemplary embodiment the retaining plug 104 can be inserted into the patient 702 and be held in place by 1. Generally the retaining plug 104 may be inserted into an incision through the patient's skin and/or other tissue prior to insertion of the objective lens 103 assembly. Alternatively, in other embodiments in accordance to other aspects of the disclosure, insertion of the objective lens assembly 103 may occur before insertion of the retaining plug 104 into the patient 702 or the objective lens 103 assembly itself may include similar structures to those of the retaining plug 104 keeping the objective lens 103 assembly from coming out of the incision. Referring back to the present exemplary embodiment, the objective lens 103 assembly can be inserted into the retaining plug 104 and retained by 2. The structural coupler 105 can be attached to the objective lens assembly 103 by 3 and can in turn be attached to the steering frame 102 by 4. Attachment of the coupler 105 to the object lens assembly 103 may occur before or after insertion of the objective lens assembly 103 into the retaining plug 104 and attachment of the coupler to the passive steering frame 102 may occur before or after the attachment of the coupler 105 to the objective lens assembly 103. The passive steering frame 102 can attach to the patient by 5. The video acquisition unit 101 can be attached to the steering frame 102 by 6 such that the video acquisition unit 101 and objective lens assembly 103 can be sufficiently optically aligned. The video acquisition unit 101 and the Display 701 can be connected by 7. The surgeon can interact with the display 701 via 8. The surgeon may then manipulate the locking assembly 107, 604, 903, 904 by 9. By the mechanism of 10 the locking assembly 107, 604, 903, 904, locks and unlocks the steering frame 102. The surgeon may also interact with the video acquisition unit 101 by 11 to adjust zoom, position, or focus parameters.

Below is a list of example compatible interactions between the components enumerated in the immediately preceding paragraph :

At interaction 1, it may be for example, mechanical interference due to the proximal flare and hexapod base and the flared distal end 402, expansion or creation of flares by inflation of distal or proximal ends of the plug, and/or friction.

At interaction 2, it may be for example, clip in to retaining feature 400, threaded together, insertion followed by a partial rotation twist to engage one or more tabs within one or more slots, a clamp, expansion of all or a portion of the plug by inflation and/or friction.

At interaction 3, it may be for example, clipping of feature 401 into a feature, such as retaining feature 400 on the plug; mating of other oppositely and permanently or temporally configured features; threading together; Friction; and/or Permanent attachment (e.g. welding, formation together as a single piece).

At interaction 4, it may be for example, mating of oppositely and permanently or temporally configured features on the two components, insertion and twisting, threading together, bolting together, and/or permanent attachment (e.g. weld, formation as a single piece)

At interaction 5, it may be for example, friction, slippery touch contact, and/or adhesive.

At interaction 6, it may be for example, clipping together, clamping one to the other, threading together (e.g. C-Mount type), insertion and twisting to engage features, and/or bolting together.

At interaction 7, it may be for example, a cable (e.g. DVI, HDMI), none (Wireless Data Communication, e.g. radio frequency, infrared).

At interaction 8, it may be for example, a touch screen, communication with another person that is controlling the display and/or optical parameters that are contributing to the information being displayed (zoom, lighting level, or the like), none (Visual observation only).

At interaction 9, it may be for example, manual manipulation of the lock activation/deactivation switch 902, foot manipulation of a remote lock activation/deactivation switch 902, manual manipulation of locks 107, manual manipulation of steering handle 903, manual manipulation of frame 102, and/or manual manipulation of stereoscopic camera 100.

At interaction 10, it may be for example, friction, mechanical interference, hydraulic, and/or pneumatic.

At interaction 11, it may be for example, manual manipulations, and/or voice commands.

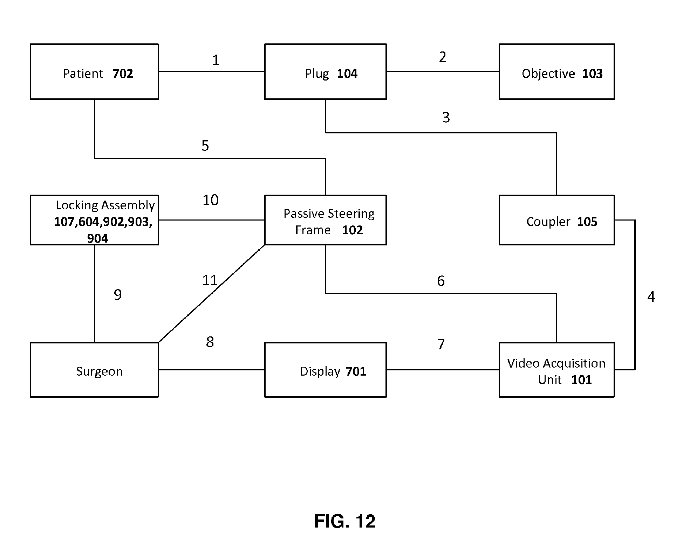

Referring now to FIG. 12, a block diagram similar to that of FIG. 11 is illustrated. In particular, it can have the same components as FIG. 11; however, the mechanical interconnections are perturbed. In the present example, the distal end of the structural coupler 105 can be directly connected to the retaining plug 104 rather than the objective 103 by 3. The proximal end can be connected to the video acquisition unit 101 directly by 4. Finally, the surgeon can steer the assembly by directly manipulating the frame 102 by 11 rather than the video acquisition unit 101. Other embodiments of the mechanical interconnections between components will be apparent to one skilled in the art upon review of the teachings set forth within.

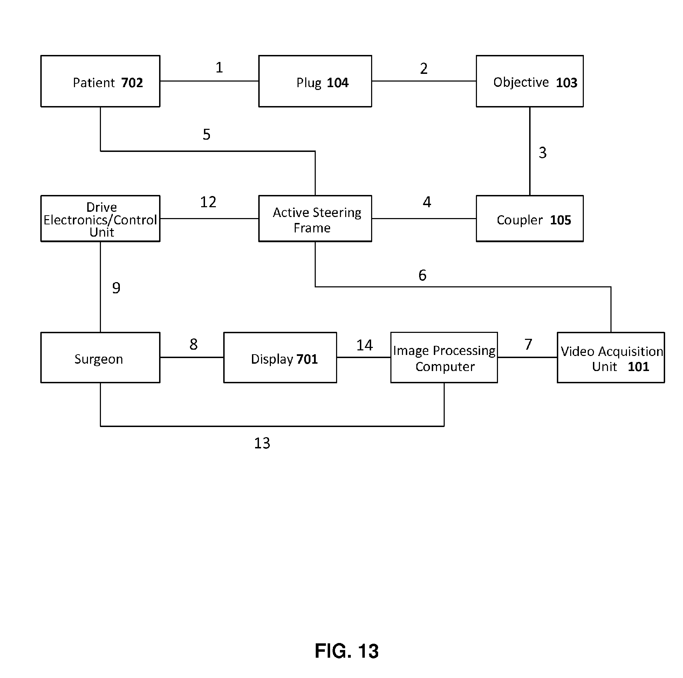

Referring now to FIG. 13, a block diagram of alternative electrical and mechanical interconnections associated with yet another exemplary embodiment of the invention is provided. The form of the block diagram is similar to that of FIG. 11 and FIG 12 but it has slightly different components and interconnections. Here the visual data coming from the image acquisition device 101 can be first sent to an image processing computer as raw data 7 before being passed to the display 701 as enhanced image data 14. The image processing computer can process the raw data in any of a variety of different ways to produce an enhanced image. For example, the image may be rotated relative to the acquisition direction to transform it to the viewing orientation of the surgeon, it may be enlarged, it may be divided into two or more images having different zooms or two or more separated images. In this configuration the image processing computer may take commands from the surgeon 11 so as to provide one or more selected views with orientations or perspectives that can be different from that originally captured by the video capture unit. A further variation in this embodiment is that the steering frame may be actively steered or manipulated by commands from a control unit (e.g. a micro-controller) wherein the joints of the struts of the frame are actuated by some mechatronic, pneumatic, or hydraulic actuators. The surgeon may give commands 9 to the control unit which produces the desired actuator commands 12 which are output to the actuators that are functionally coupled to the active steering frame. In some variations of this embodiment, the control unit and image processing computer may be the same machine. Furthermore, the human machine interface producing signals 9 and 13 could be the same or separate units (e.g. keyboard, joy stick, mouse, touch screen, microphone, or combination thereof). In some embodiments, it may be a joystick or touch controls that are integrated in the laparoscopic instruments so the surgeon would not have to let go of the instruments to make visualization adjustments. However it may be useful to have a separate human machine interface (HMI) as the instruments can be switched many times for some procedures.

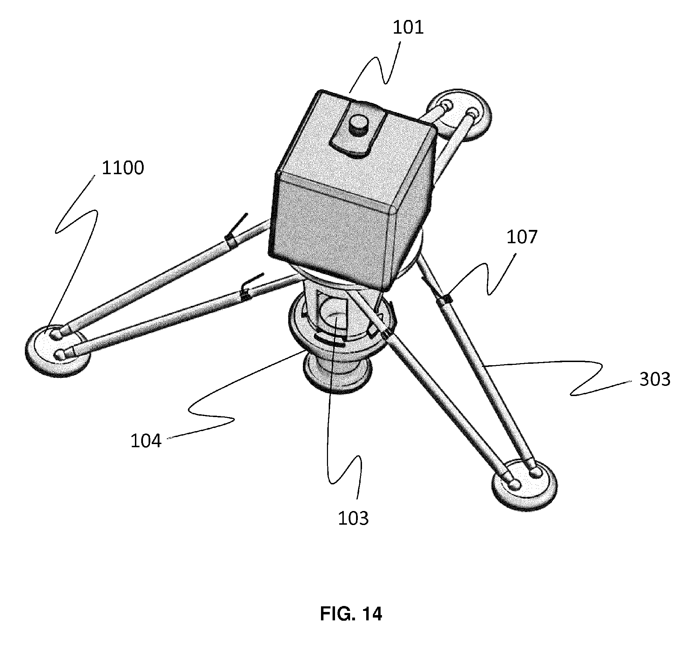

Referring now to FIG. 14, a perspective view of an alternative steering frame configuration 102 where the base plate 108, which attached all of the struts structurally, is replaced by pads 1100 that connect only pairs the distal ends of the struts (i.e. arms of adjustable length) together is illustrated. Of course in other alternative embodiments within the scope of the disclosure, different numbers of struts could be joined by the pads or each arm could be connected to its own pad. The pads in turn may be attached/supported by the patient and/or table. In the case of patient attachment, the pads may be attached by an adhesive, suction, friction, suture or pinching mechanism. In the case of the table the pads could be removable or permanent. Removable pads could be attached by bolting, clamping, adhesive, friction or suction. Permanently attached pads could be attached by the same means or, for example, by welding, soldering, or brazing. In this manner the struts 303 of the manipulator can be structurally fixed and stabilized by the patient, ceiling, and/or the table.

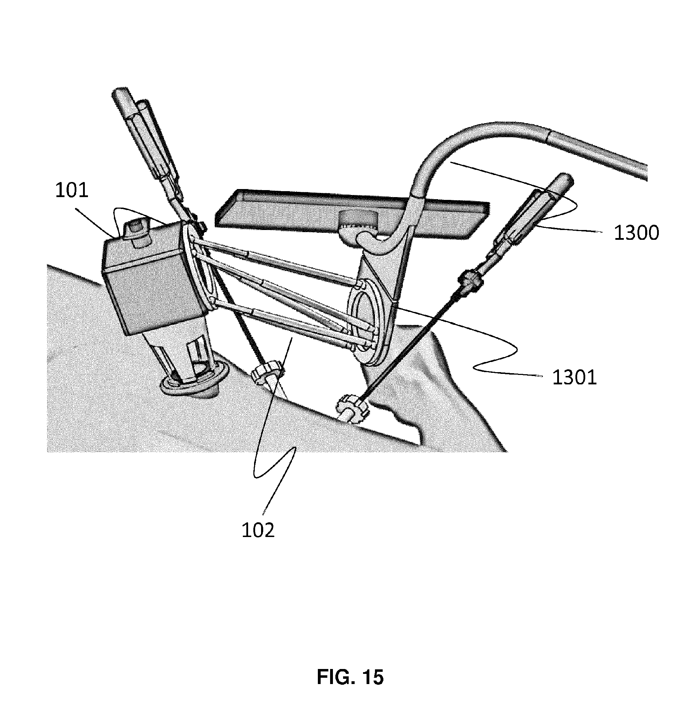

Referring now to FIG. 15, another exemplary configuration of the system where the steering frame 102 can be attached to an external support device 1300 and to a side of the video acquisition unit 101 is illustrated. Additional degrees of freedom may be added to the steering frame 102, for example, by adding joints 1301 to the external support device 1300, allowing for alignment with the patient.

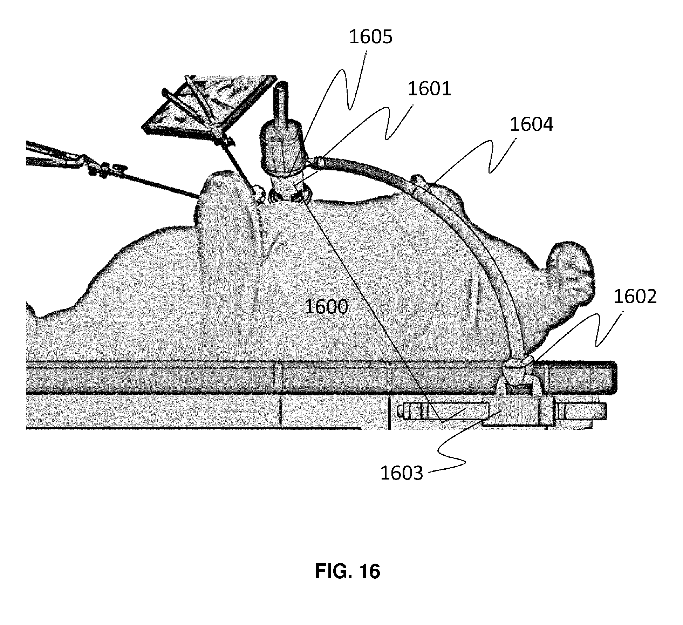

Referring now to FIG. 16, yet another exemplary embodiment of the steering frame where it is in the form of a serial linked arm 1600 mounted to a rigid location in the surgical theater rather than sitting directly on the patient is illustrated. A convenient mounting location for the support arm can be the surgical table 1603. In this embodiment, a prismatic joint arm 1604 can be attached to the camera by a rotational joint 1601 and the surgical table mount 1603 by a rotational joint 1602. The prismatic arm can be straight or circular in order to avoid conflicting with the body of the patient. The rotational joint 1602 can be of the spherical type or universal type, although universal may be preferred as may ensure that the arm can stay arced over the patient. The rotational joint 1601 at the imaging device can be spherical, universal or a combination of standard universal and rotational joints to achieve the necessary degrees of freedom. As with the embodiment of FIG. 1, the frame of this embodiment may be preferably passive with a locked and free mode though in other alternatives it may be an active device. During usage, it may be necessary to lock all or only some of the joints to hold the imaging device in place. For example, the prismatic and rotational joint at the table mount may lock while the rotational joint at the imaging device stays unlocked, allowing the imaging device to rotate with patient movement. There may also exist another joint 1605 which is rotational about the optical axis of the camera, prismatic about the optical axis of the camera or both. The rotational aspect may be used to compensate for viewing orientation. The prismatic aspect may be used to compensate for reduced/increased insufflation while ensuring and unchanged optical axis orientation.

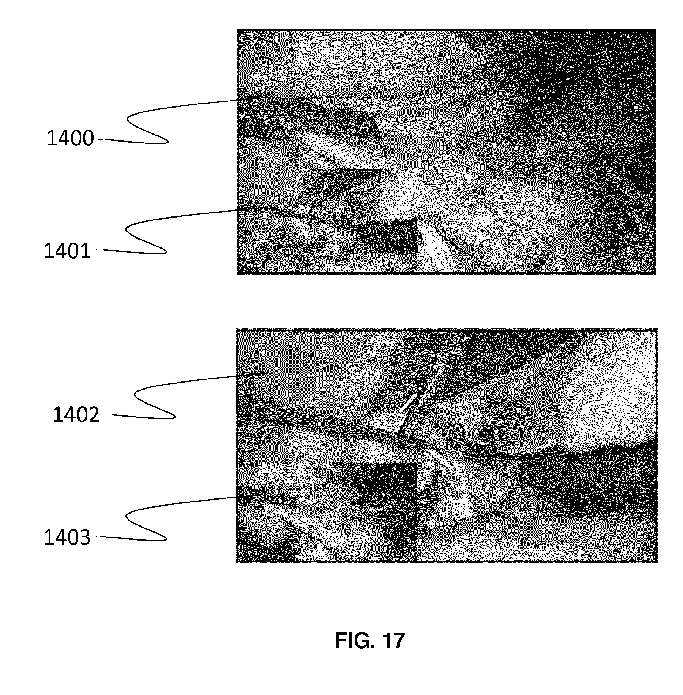

Referring now to FIGS. 17A and 17B, two picture-in-picture schemes to show wide angle and zoomed views simultaneously are depicted. In some procedures, a wide angle and zoomed view of the surgical field may be available simultaneously by means of an inlaid image. The wide angle image 1401 may be inlaid on the full size zoomed view 1400 as shown in FIG. 17A, or the zoomed image 1402 may be inlaid on the full screen wide angle view 1403 as shown in FIG. 17B. The zoomed view may be achieved by digitally cropping and resizing the image (lowering resolution) or optically. In the optical zoom case, it may be that the image is 2D and one pupil can be used for zoom while the other can be capturing the wide angle view. If 3D is desired then added optics may be necessary, for example, two optical channels for any 3D views and one optical channel for any 2D view. For 3D views of both the full screen image and the inlaid image could require four pupils.

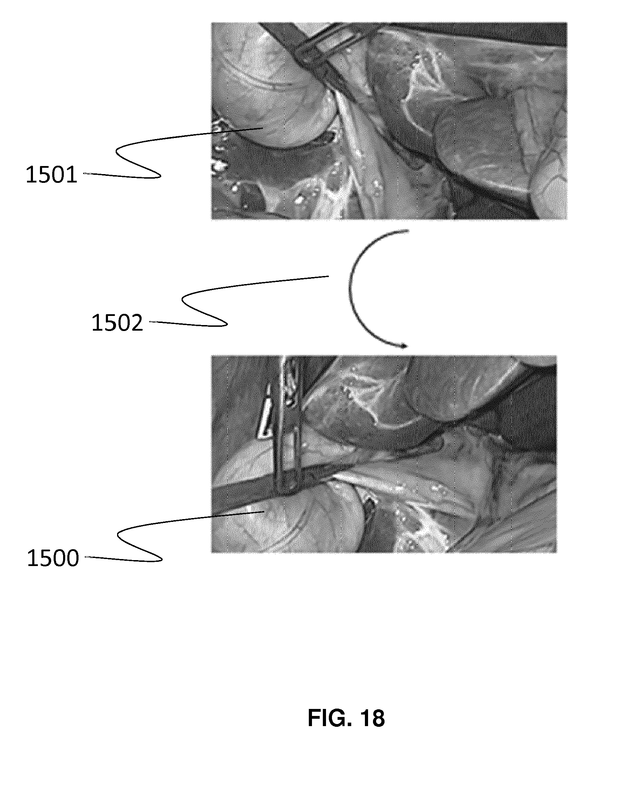

Referring now to FIG. 18, images showing how image processing can be used to provide the surgeon with enhanced and more natural/intuitive views of the surgical area even when the camera is not viewing the area from the same direction as the surgeon by manipulating the image to compensate for misalignment of the camera are illustrated. The ideal view realignment would render the image as if it was viewed by the line of sight of the surgeon. A simple example of this type of image processing compensation would be to adjust the rotational displacement of the camera. If image 1501 (i.e. the upper image) is from the perspective of the camera with a 45 degree rotational misalignment relative to the surgeon, applying a digital rotation 1502 to the image can render the correct surgical view 1500 (i.e. the lower image). This may be controlled by a user interface, or the compensation could occur as a result of signals being sent to the computer by sensors such as tilt sensors, distance sensors, encoders, accelerometers or gyroscopes. A more complex compensation would be to construct a dense depth map of the scene using two view computer vision techniques, since each pupil of the stereoscopic imaging system gives two distinct views of the surgical field. This information could then be used to reproject the anatomy on a virtual camera collocated along the line of sight of the surgeon.

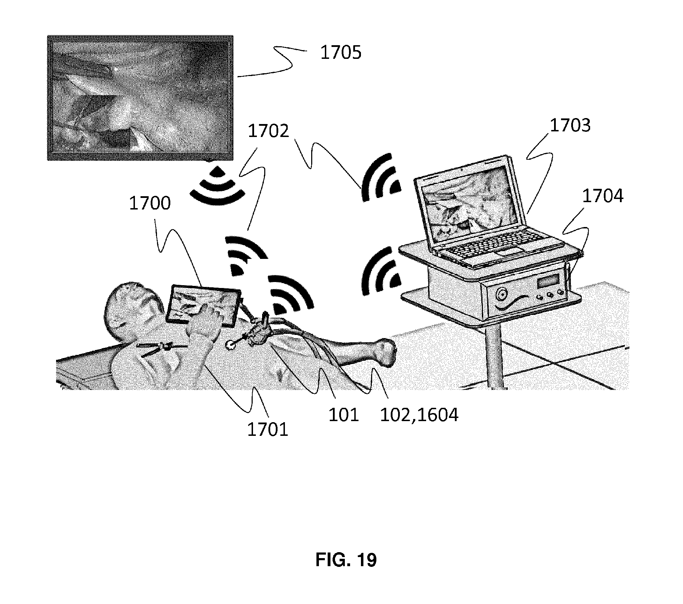

Referring now to FIG. 19, an illustration showing how peripheral devices in the operating theater can be controlled using a touch screen computer display 1700 is presented. In particular, how the surgeon 1701 may interface with the computer using typical touch screen interactions. Since the touch screen computer display 1700 can have communication hardware, it may control or send data to a plurality of devices both wirelessly 1702 and/or wired. The image acquisition unit 101 may receive controls pertaining to independent left and right zoom levels. The standalone computer 1703 may receive live streaming video data for patient records, computational analysis, or the like. Peripheral surgical equipment 1704 may also be controlled such as insufflation, cauterization power or irrigation. Electronically enabled utilities may be controlled such as lighting, room temperature.

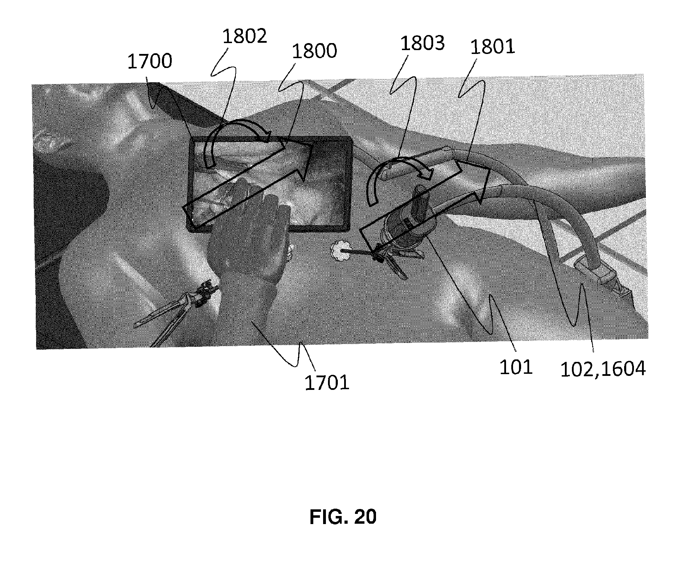

Referring now to FIG. 20, a representation of how a touch screen computer display 1700 in communication with an active steering arm 102, 1604 may allow the surgeon to control the camera 101 is presented. In particular, how touch screen gestures can allow the surgeon to pan and rotate the view of the surgical field. In some embodiments, for example, to pan the view the surgeon 1701 can swipe one or a plurality of fingers across the touch screen computer display 1700 actuating the steering arm 102,1604 in such a manner that the camera tilts to provide a view that mimics the dragging surgeon's fingers. In addition or alternatively, the surgeon can rotate the view by rotating while dragging two or a plurality of figures on the touch screen computer display 1700 actuating the steering arm 102,1604 in such a manner that the camera rotates to provide a view that mimics the rotation of the surgeon's 1701 fingers. In another embodiment the pan and rotate can be accomplished by soft buttons. In yet another embodiment the pan and rotate is accomplished by a hardware interface. Further, some embodiments, may include more than one control mechanism with one control mechanism overwriting the instructions of the other when in conflict.

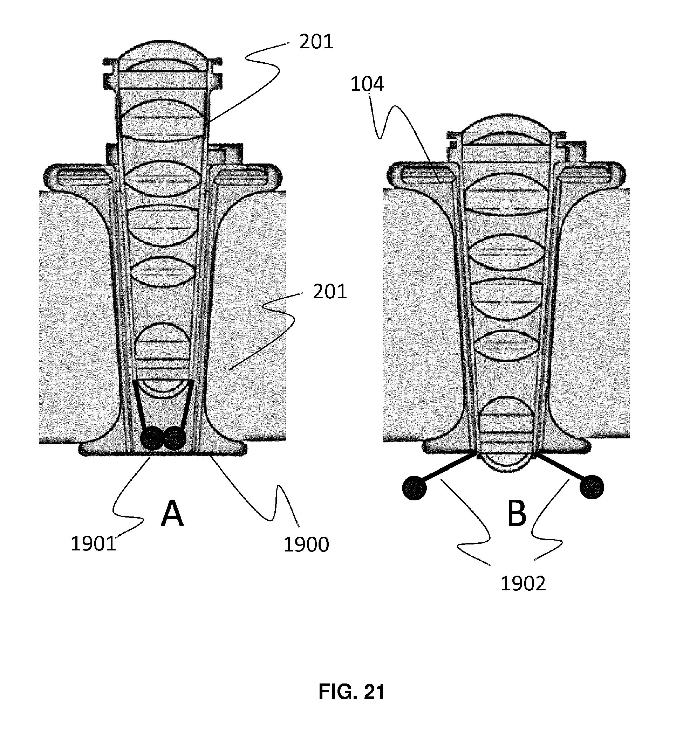

Referring now to FIG 21A and FIG 21B, representations of how an illumination device 1900 can be inserted through a channel in a collapsed 1901 configuration such that when entering the surgical cavity the illumination device 1900 unfurls to an extended configuration 1902 are presented. In an exemplary embodiment, the illumination device 1900 can be attached to the objective lens assembly 201 in a collapsed configuration 1901. After the illumination device 1900 has passed through the retaining plug 104 it can unfurls to the extended configuration 1902. In another exemplary embodiment the illumination device 1900 may be attached to the retaining plug 104. As previously mentioned, the illumination device 1900 may comprise LEDs to provide the illumination.

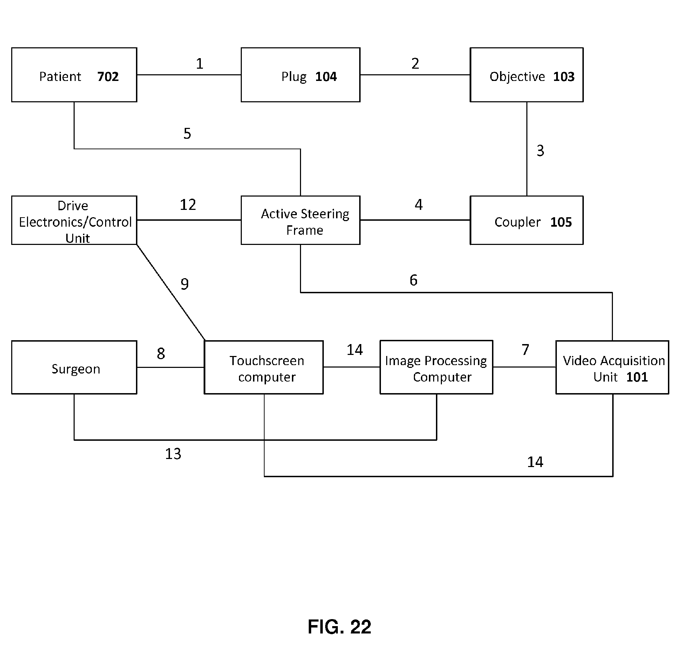

Referring now to FIG 22, a block diagram representing the interconnections between components is depicted. It is similar to FIG 13; however, the drive electronics to the steering arm are taking commands from the touch screen computer display 1700 across connection 9. This connection may be a wireless connection or a hardwire connection. Similarly the image acquisition device may be connected to the touch screen computer to allow for communication to change each optical channel's zoom level.

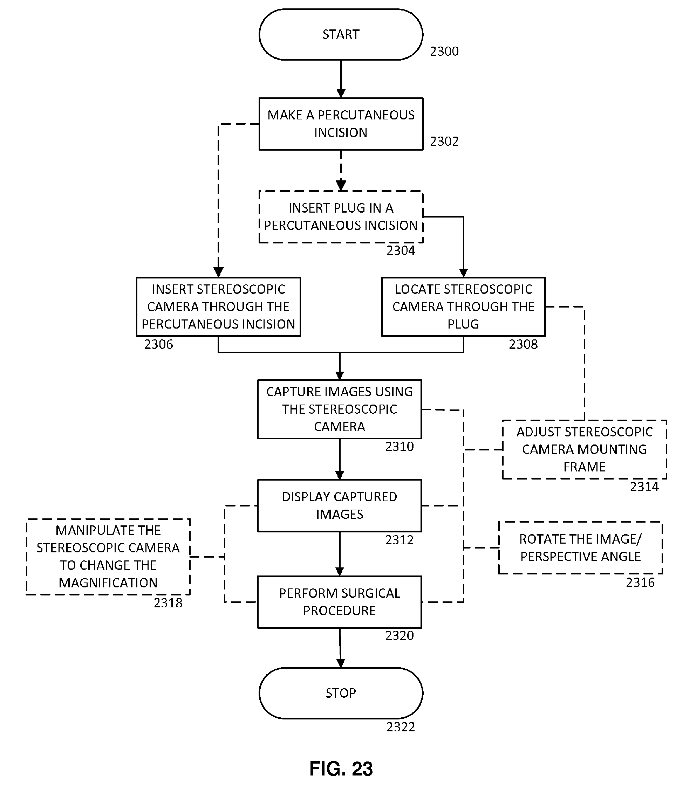

Referring now to FIG. 23, a flowchart illustrating exemplary method steps that can be implemented according to aspects of the present disclosure are shown. Beginning at step 2300, sterilization and/or any other commonly known and performed routine to begin a MIS procedure may occur. Subsequently, at step 2302, a percutaneous incision in the skin of a patient can be made. As previously described, the incision may be made around an area where the MIS procedure will take place. The size of the percutaneous incision can be so that a retaining plug can be tightly inserted through the skin.

In some embodiments of the system, optionally at step 2304, a retaining plug can be inserted through the percutaneous incision. The functional purpose of the retaining plug 104 can include holding the device down to the patient by an expanded flange. Some plugs may be deformable enough to allow insertion into the incision, either by the natural compliance of the material that it is constructed from, by being or having inflatable components, or having articulating components. In some embodiments the plug may be disposable, but at the minimum it should be sterilizable. Alternatively, in some embodiments, the objective of the stereoscopic camera may include structural features or articulating components capable of holding the stereoscopic camera onto the patient. In these types of embodiments, at step 2306, at least a portion of the objective forming part of the stereoscopic camera may be inserted through the percutaneous incision without the need of a retaining plug. The objective lens or lens assembly 103 being inserted may be made of glass or plastic, however in some preferred embodiments it can be disposable, but at the minimum it should be sterilizable.

In embodiments where a retaining plug is used, at step 2308, at least a portion of the stereoscopic camera can be located through the retaining plug. In some embodiments, the portion may be the objective of the stereoscopic camera for the purposes described throughout the disclosure. Further, because the stereoscopic camera forming part of the video acquisition unit can contains numerous optical and electronic components of the system which may limit the ability for this unit to be treated as disposable, it can be designed for multiple uses and the unit may be configured for ease of surface sterilizability or encapsulation by a disposable biocompatible encapsulating material.

At step 2310, images can be captures using the stereoscopic camera. Processing of the captured images can then occur for a processor to display the captured images at step 2312. For example as previously presented, the display 701 can communicate with the Video Acquisition Unit 101 by 7. This could be a single direction communication where the image data may be simply sent to the display 701 for viewing. The display however may also have touch screen controls for zoom, focus, image freezing, or other camera mode selections, requiring 7 to support two-way information flow. A touch screen interface could be button based or gesture based. For example, a gesture to zoom out would be to perform a two finger pinching motion on the screen and the picture-in-picture roles could be reversed by swiping from the smaller image to the center of the screen. The display 701 may support VGA resolution (640x480) all the way up to true high definition (1920x1080p) or beyond. Since the video acquisition unit 101 is stereoscopic, the display 701 preferably supports either active or passive 3D display technology. In the some embodiments, the display is autostereoscopic (e.g. parallax barrier), requiring no glasses for viewing a 3-D effect.

Referring back to FIG. 23, at step 2320, the MIS procedure can then be performed by the practitioner utilizing the stereoscopic system. At a point prior to, during, and/or after steps 2318, 2314, and/or 2316 may take place as it may be appropriate. However, one or more of these steps may not occur depending on the type of MIS procedure, and the settings and configuration of the embodiment being implemented.

At step 2314, the stereoscopic camera mounting frame can be adjusted. As previously described, the structural coupler 105 must be rigid enough to maintain sufficient optical alignment between the objective 103 and the video acquisition unit 101. It must have means to attach to the objective 103 or retaining plug 104 and means to attach to the steering frame 102 or video acquisition unit 101. For example, in some embodiments, the passive steering frame 102 can be a mechanism with a fixed base, a movable end effector, and a linkage system with struts and joints that connect the two. The frame may include a normal state or at least a settable state such that if the joints are locked, the end effector cannot move relative to the fixed base but when not locked the base and movable end effector can be easily and quickly manually reoriented with respect to one another. Therefore by disengaging the locking mechanism, causing relative movement, and then engaging the locking mechanism, the surgeon can move the end effector to the desired position and fix it into the new position thus maintaining the new position of the end effector and any device attached to it. In the context of at least some embodiments of the current invention, the passive steering frame can be a parallel joint passive steering frame with a base fixed on the patient and the stereoscopic video camera 100 attached to the end effector. As a parallel manipulator, the frame 102 can have as little as three parallel joints and as many as six. Any number above six may be redundant from a locking perspective, but may be useful for other purposes. These additional joints might provide for measuring position, limiting motion, strength, or changing the frame's dynamic properties such as damping. In some implementations, less than six joints may be tolerated since some degrees of freedom may be limited by the insertion of the retaining plug 104 and objective 103 into the surgical incision.

The locking assembly 107-604-902-903-904, for example, in its simplest design must allow the user to lock the end effector relative to the fixed base by immobilizing a finite number of joints. In an exemplary embodiment, the surgeon can manipulate a single switch that can in turn engage and disengage the locks at each joint simultaneously. The switch needs a minimum of two positions. One associated with an engaged lock and one associated with a disengaged lock. If the lock is purely mechanical or pneumatic, then the force required to disengage or engage the lock comes from the user manipulating this switch. The means of power transmission from the switch to the lock must account for the articulation of the joints between the switch and the lock (e.g. flexible pneumatic tubes, cable in axially stiff sheath). The lock must then interfere with the relative movement of the joints through some locking means (e.g. friction, component interference, hydrolocking, magnetorheologic modulation, jamming, electrical or magnet clamping, or the like).

In some embodiments, the movement of the objective lens assembly may be largely rotational in nature such that the objective lens assembly pivots about the most distal lens or about the entry point of the assembly into the skin or other tissue of the patent. In other embodiments, movement of the assembly may be such that it undergoes some translation relative to the base and as such some repositioning of the base relative to the patient's skin may be used to ensure that undue stressing of the patient's tissue does not occur.

As described herein, each strut may include two elements that slide relative to each other giving an adjustability that is limited by something less than ½ the maximum length of each strut. In some alternative embodiments, the struts may have more than a single extending element (e.g. two or more telescoping segments with each having its own lock such that multi-stage extension can occur thus improving the maneuverability of the passive steering system. In those embodiments, all locks may still be engaged or disengaged simultaneously as it does not matter which segments undergo relative movements so long as the final desired positioning can be achieved. In other multi-stage embodiments, only some of the locks may be disengaged at any given time.

At step 2316, the image/perspective angle may be rotated as previously described. For example, the image may be rotated relative to the acquisition direction to transform it to the viewing orientation of the surgeon. The image processing computer may take commands from the surgeon so as to provide one or more selected views with orientations or perspectives that can be different from that originally captured by the video capture unit.

At step 2318, the stereoscopic camera and/or an associated component can be manipulated to change the magnification. For example, the video acquisition unit can typically include optical zoom and focusing mechanisms, photosensitive integrated circuits 204, and digital image processing electronics which can be manipulated/adjusted. Moreover, in some embodiments, two photosensitive integrated circuits, one associated with each pupil, and thus with each optical channel can be created by the two stereoscopic pupils 203, may be the extent of the electronic components in the unit. However, to get better image quality and truer color, 3 or 4 photosensitive integrated circuits may be used to sense different wavelengths of light separately (e.g. red, green, and blue). In this case, extra optical hardware may need to be added, such as dichroic prisms, in order to optically separate the different wavelengths of light. In still other embodiment variations, it may be desirable to sacrifice image quality for compactness, and use a single photo sensor to capture both right and left images, half for the left and half for the right. Zooming could be continuous, or could have a finite number of discrete zoom levels. Focus could be manual or automatic.

At step 2322, after the MIS procedure is finished, the plug and/or stereoscopic camera may be removed from the percutaneous incision. It is to be understood that an additional number of steps can occur depending on the embodiments as well as the type of MIS procedure. MIS procedures can include, for example, in the areas of thoracoscopic, laparoscopic, pelviscopic, arthroscopic surgeries. For laparoscopic surgeries for example, significant utility will be found in cholecystectomy, hernia repair, bariatric procedures (bypass, banding, sleeve, or the like), bowel resection, hysterectomy, appendectomy, gastric/anti-reflux procedures, and nephrectomy.

The many features and advantages of the invention are apparent from the detailed specification, and thus, it is intended by the appended claims to cover all such features and advantages of the invention which fall within the true spirit and scope of the invention. Further, because numerous modifications and variations will readily occur to those skilled in the art, it is not desired to limit the invention to the exact construction and operation illustrated and described, and accordingly, all suitable modifications and equivalents may be resorted to, falling within the scope of the invention.

## Claims

1. A method for viewing a surgical area of a minimally invasive surgical procedure, comprising:
capturing an image of an internal surgical area via an image capturing device including at least two optical paths, at least one objective lens assembly including retaining structures useful to hold the objective lens assembly inside a percutaneous incision, and an end effector forming part of a frame supporting the image capturing device;
configuring said end effector to be movably coupled with respect to the internal surgical area;
further configuring a locking mechanism to lock said end effector at a desired position relative to the internal surgical area;
transmitting said captured image to a display positioned to be viewed by a practitioner during a minimally invasive surgery.

2. The method of claim 1, wherein the at least one objective lens assembly is inserted into a retaining plug positioned through the percutaneous incision.

3. The method of claim 1,
wherein the objective lens assembly comprises a proximal end, a distal end, and one or more optical lenses in between, the objective lens assembly is inserted at least partially into the percutaneous incision, and
wherein said objective lens assembly is configured to be placed such that the proximal end is outside of the patient's body while the distal end is disposed inside of the body cavity.

4. The method according to any preceding claim, further comprising processing a practitioner's input to modify the image being displayed.

5. The method according to any preceding claim, wherein the display is positioned in a sterile field.

6. The method according to any preceding claim, wherein the display is a touch screen display being used to display an image and further as a user interface.

7. The method of claim 6, additionally comprising:
modifying one or more of: the field of view of said image capturing device, the magnification, picture properties, and number of views displayed based on a practitioner's input on said touch screen display.

8. The method according to any preceding claim, wherein the locking mechanism includes one or more of a mechanical, pneumatic, and electrical locking mechanism.

9. The method according to any preceding claim, further comprising:
configuring the end effector to be automated in order to control a viewpoint of the image capturing device.

10. The method according to any of preceding claims 1-9, wherein said end effector is movably coupled with respect to the internal surgical area by extending or shortening a plurality of struts.

11. The method according to any of preceding claims 1-9, wherein said end effector is movably coupled with respect to the internal surgical area via at least one extendable/retractable arm having a rotatable coupling that functionally connects said arm to the base and to the end effector.

12. The method according to any of preceding claims 1-9, wherein said end effector is movably coupled with respect to the internal surgical area via a movable pivotal joint that functionally connects said arm to the base and to the end effector.

13. The method of claim 2, further comprising:
configuring one or both of said retaining plug and objective lens assembly to include a light source capable of illuminating the internal surgical area.

14. The method according to any preceding claim, further comprising:
processing said captured image to provide a stereoscopic image.
